# EUROPEAN PATENT APPLICATION

(11) **EP 4 477 235 A1**
(43) Date of publication of application: **18.12.2024**
(21) Application number: 23179525.3
(22) Date of filing: 15.06.2023
(51) Int. Cl.: A61K 45/06, A61P 9/06, A61P 31/14

(54) **DIAGNOSTIC AND THERAPEUTIC APPLICATIONS OF CARDIOPULMONARY INTERFACES IN THE STELLATE GANGLION**

(71) Applicant: Technische Universität München, 80333 München (DE)
(72) Inventor: The designation of the inventor has not yet been filed
(74) Representative: Weickmann & Weickmann PartmbB

(57) **Abstract**

The present invention relates the use of an anti-inflammatory agent for inhibiting and/or preventing a cardiovascular complication in a subject suffering from a respiratory disease. The present invention also relates to the use of an anti-inflammatory agent for preserving cardiac innervation in a subject suffering from respiratory disease.

## Description

The present invention relates to the use of an agent selected from the group consisting of an anti-inflammatory agent, a neuroprotective agent, and a cell death-preventing agent for preventing and/or treating cardiovascular dysfunction in a subject suffering from a respiratory disease. The present invention also relates to the use of an anti-inflammatory agent for preserving cardiac innervation in a subject suffering from respiratory disease.

### BACKGROUND

The intricate interplay between respiratory diseases, specifically pneumonia, and cardiovascular complications, such as arrhythmias, remains an enigma in the field of medicine. While pneumonia primarily affects the lungs, mounting evidence suggests that it can exert profound effects on the cardiovascular system (Bartlett, Ludewick, Lee, & Dwivedi, 2019; Bartlett et al., 2022; Corrales-Medina, Musher, Shachkina, & Chirinos, 2013; Corrales-Medina et al., 2012, 2011; Lee, Ho, Wang, Leong, & Wei, 2022; Stotts, Corrales-Medina, & Rayner, 2023; Thomson & Rustein, 1946). The association between lung disease and cardiac abnormalities, particularly arrhythmias, has intrigued researchers, yet the underlying mechanisms and causal relationships have remained elusive (Corrales-Medina, Musher, Shachkina, & Chirinos, 2013; Corrales-Medina et al., 2012; Stotts, Corrales-Medina, & Rayner, 2023; Pletz et al., 2022).

Especially during the worldwide COVID pandemic, many observational studies reported cardiovascular complications indicated by myocardial infarction, myocarditis, venous thromboembolism, and arrhythmias. Up to 18% of SARS-CoV2 positive patients presented with arrhythmias, whereas the majority had no previous cardiac disease. The most common manifestations of arrhythmias were sinus tachycardia, palpitations, atrial arrhythmias as well as bradycardic arrhythmias, although the distribution varies from country to country (Azevedo et al., 2021).

Understanding this perplexing link is crucial for unraveling the complexities of disease progression, identifying potential therapeutic targets, and enhancing patient outcomes.

Co-pending application PCT/EP2023/065279, the content of which is herein incorporated by reference, discloses the use of an anti-inflammatory agent for inhibiting and/or preventing damage of a peripheral ganglion in a subject, particularly in a subject suffering from a cardiac disease, or for preserving cardiac innervation in a subject suffering from cardiac disease.

### SUMMARY OF THE INVENTION

It was an object of the invention to inhibit and/or reduce the occurrence of cardiovascular dysfunction in subjects suffering from a respiratory disease, particularly from pneumonia. It was a further object of the invention to preserve cardiac innervation in a subject suffering from a respiratory disease, particularly from pneumonia. It was still a further object of the invention to identify a subject in risk of the occurrence of cardiovascular dysfunction.

The present inventors have identified coincidence of a marked injury of lung-innervating axons with macrophage accumulation and increased neuronal activity in heart-innervating sympathetic neurons of the stellate ganglion.

A first aspect of the present invention is an agent selected from the group consisting of an anti-inflammatory agent, a neuroprotective agent, and a cell death-preventing agent for use in a method for preventing and/or treating cardiovascular dysfunction in a subject suffering from a respiratory disease.

A further aspect of the present invention is a method for preventing and/or treating cardiovascular dysfunction in a subject suffering from a respiratory disease, comprising administering an effective amount of an agent selected from the group consisting of an anti-inflammatory agent, a neuroprotective agent, and a cell death-preventing agent to a subject in need thereof.

A further aspect of the present invention is the use of an agent selected from the group consisting of an anti-inflammatory agent, a neuroprotective agent, and a cell death-preventing agent for the manufacture of a medicament for preventing and/or treating cardiovascular dysfunction in a subject suffering from a respiratory disease.

A further aspect of the present invention is a method for identifying a subject being at risk of a cardiovascular dysfunction, comprising determining at least one parameter associated with the function of the stellate ganglion.

A further aspect of the present invention is a compound which is a modulator of a biomarker selected from a gene which is characterized by an altered, e.g., increased, or decreased expression level associated with damage or risk of damage in the stellate ganglion, for use in a method for preventing and/or treating cardiac dysfunction in a subject.

### PREFERRED EMBODIMENTS

In the following, preferred embodiments of the specification are listed:
1. An agent selected from the group consisting of an anti-inflammatory agent, a neuroprotective agent, and a cell death-preventing agent for use in a method for preventing and/or treating cardiovascular dysfunction in a subject suffering from a respiratory disease.
2. The agent of embodiment 1 for the use of embodiment 1, wherein the cardiovascular dysfunction is selected from acute coronary syndrome, myocardial infarction, heart failure, endocarditis, myocarditis, pericarditis, venous thromboembolism, arrhythmia, and blood pressure fluctuations such as hypotension and/or pulmonary hypertension.
3. The agent of embodiment 1 for the use of embodiment 1 or 2, wherein the cardiovascular dysfunction comprises arrhythmia.
4. The agent of embodiment 1 for the use of any one of embodiments 1-3, wherein the agent inhibits and/or prevents damage in the stellate ganglion of the subject.
5. The agent of embodiment 1 for the use of any one of embodiments 1-4, wherein the agent inhibits and/or prevents degeneration and/or death of cells in the stellate ganglion, particularly degeneration and/or death of neuronal cells.
6. The agent of embodiment 1 for the use of any one of embodiments 1-5, wherein the subject is suffering from a respiratory disorder selected from acute respiratory distress syndrome (ARDS), pneumonia, chronic bronchitis, or tuberculosis.
7. The agent of embodiment 1 for the use of any one of embodiments 1-6, wherein the subject is suffering from pathogen-associated respiratory disorder including virus- or bacteria-associated ARDS or pneumonia, particularly from coronavirus-associated ARDS or pneumonia.
8. The agent of embodiment 1 for the use of any one of embodiments 1-6, wherein the subject is suffering from non-pathogen-associated respiratory disorder including chronic obstructive pulmonary disease (COPD), asthma, or pulmonary fibrosis.
9. The agent of embodiment 1 for the use of any one of embodiments 1-8, wherein the subject is suffering from an acute form of the respiratory disorder.
10. The agent of embodiment 1 for the use of any one of embodiments 1-9, wherein the agent is administered as a preventive intervention.
11. The agent of embodiment 1 for the use of any one of embodiments 1-10, wherein the agent is administered when the stellate ganglion is still substantially intact.
12. The agent of embodiment 1 for the use any one of embodiments 1-11, wherein the agent is administered when the stellate ganglion is characterized by the absence of ganglion hypertrophy, disrupted ganglion activity and/or ganglion inflammation.
13. The agent of embodiment 1 for the use of any one of embodiments 1-12, wherein the subject is identified as being at risk of damage of the stellate ganglion.
14. The agent of embodiment 1 for the use of embodiment 13, wherein the risk of damage of the stellate ganglion is identified by determining at least one parameter selected from periodic repolarization dynamics, e.g., as assessed by a 3D high-resolution resting ECG, ganglion size, e.g., SCG size, e.g., as assessed by ultrasound, a biomarker associated with respiratory disease including but not limited to blood levels of inflammatory markers (e.g. CRP, IL-6), pulmonary function tests, blood gas analysis, sputum analysis, pulmonary imaging (CT scan, MRI imaging with contrast enhancement to image inflammation), a biomarker associated with cardiac disease including but not limited to ANP, BNP, cTnT and its derivatives and/or a biomarker specifically associated with damage of the stellate ganglion.
15. The agent of embodiment 1 for the use of any one of embodiments 1-14, wherein the agent is administered in response to an observed damage and/or dysfunction of the stellate ganglion.
16. The agent of embodiment 1 for the use of any one of embodiments 1-15, wherein the agent is locally administered to the stellate ganglion, e.g., by injection.
17. The agent of embodiment 1 for the use of any one of embodiments 1-16, which is an anti-inflammatory agent.
18. The agent of embodiment 17 for the use of any one of embodiments 1-16,
   wherein the anti-inflammatory agent is selected from:
   - a glucocorticoid, including but not limited to Dexamethasone;
   - an immunoglobulin preparation, including but not limited to IVIg;
   - a complement inhibitor, including but not limited to a C3 antagonist such as Compstatin or Pegcetacoplan, or a C5 antagonist such as Eculizumab, Zilucoplan, Tesidolumab, or SKY59;
   - a nucleic acid therapeutic including but not limited to anti-miR21;
   - an immunomodulator, including but not limited to Alemtuzumab, Mitoxantron, or Orcrelizumab;
   - an antagonist of a chemokine, cytokine or colony-stimulating factor including but not limited to Etanercept, Adalimumab or PLX5622;
   - a macrophage inhibitor including but not limited to a bisphosphonate compound such as clodronate;
   - a NLRP3 inflammasome inhibitor including but not limited to MCC950, Glyburide, Partehnolide, or β-OH-Butyrate; and
   - a CSF1R inhibitor including but not limited to Pexidartinib.
19. The agent of embodiment 1 for the use of any one of embodiments 1-16, which is a neuroprotective agent.
20. The agent of embodiment 19 for the use of any one of embodiments 1-16,
   wherein the neuroprotective agent is selected from:
   - a free radical scavenger including but not limited to NXY-059, PEG-SOD, Tempol, hydroxystilbene, and oxyresveratrol;
   - a nerve growth fand/or neurotrophic factor including but not limited to an insulin-like growth factor (IGF), nerve growth factor (NGF) and a brain-derived neurotrophic factor (BDNF);
   - a mitochondrial enhancer including but not limited to Carnitine, Resveratrol, PQQ and alpha-lipoic acid;
   - a nucleic acid therapeutic; and
   - a statin including but not limited to Atorvastatin, Mevastatin, Rosuvastatin and Simvastatin.
21. The agent of embodiment 1 for the use of any one of embodiments 1-16, which is a cell death-preventing agent.
22. The agent of embodiment 21 for the use of any one of embodiments 1-16,
   wherein the cell death-preventing agent is selected from:
   - a Bcl-2 family modulator including but not limited to Venetoclax, Navitoclax, Obatoclax and ABT-199;
   - a nucleic acid therapeutic; and
   - a caspase inhibitor including but not limited to Z-VAD-FMK, Q-VD-OPh, Emricasan, VX-765 and Z-DEVD.
23. The agent of any one of embodiments 1 or 17-22 for the use of any one of embodiments 1-16, wherein the subject is a human subject.
24. A method for identifying a subject being at risk of a cardiovascular dysfunction, comprising determining at least one parameter associated with the function of the stellate ganglion.
25. The method of embodiment 24 wherein the subject suffers from a respiratory disease.
26. The method of embodiment 24 or 25, wherein the parameter is selected from a physical parameter such as periodic repolarization dynamics and ganglion size, and/or a physiological parameter such as ganglion function, inflammation and/or a biomarker, a biomarker generally associated with a cardiac disease, and/or a biomarker specifically associated with damage of the stellate ganglion.
27.A compound which is a modulator of a biomarker selected from a gene which is characterized by an altered, e.g., increased, or decreased expression level associated with damage or risk of damage in the stellate ganglion, for use in a method for preventing and/or treating cardiac dysfunction in a subject.
28. The compound of embodiment 27 for the use of embodiment 27, wherein the subject suffers from a respiratory disease.
29. The compound of embodiment 27 for the use of embodiment 27 or 28,
   (i) which is an agonist of the biomarker or an antagonist of the biomarker, and/or
   (ii) which is selected from an antibody or an antigen-binding fragment thereof and a nucleic acid molecule, e.g., an mRNA molecule, an antisense molecule, or a siRNA molecule, and/or
   (iii) which is a genome editing compound, such as CRISP/Cas, a zinc finger nuclease (ZFN), or a transcription activator-like effector nuclease (TALEN), preferably a CRISPRi/dCas9 system for selective gene silencing.

### DETAILED DESCRIPTION

The present inventors investigated whether local pulmonary or cardiac sympathetic innervation is affected in patients suffering from pneumonia by characterizing the alterations of neuronal circuits in pneumonia in the stellate ganglion which provides sympathetic innervation to both, the lung (Herring et al., 2019) and the heart (V6gh et al., 2016).

Histomorphologic analysis of the stellate ganglia where the axons originate uncovered substantial macrophage accumulation. Single nuclei RNA sequencing of the latter furthermore showed distinct neuronal dysfunction. Using spatial RNA sequencing, heart- and lung-innervating neurons could be located and were found to be in direct proximity to each other, suggesting a mechanism for the spread of the disease between these organs. Analysis of sympathetic activity indicated by periodic repolarization dynamics in a prospective clinical study confirmed increased sympathetic activity.

Together, these results indicate severe inflammation and/or dysfunction in stellate ganglion neurons that also control cardiac rhythmicity. This provides evidence for a substantial sympathetic involvement during pneumonia that might be causally linked to cardiac arrhythmia and reveals the defective sympathetic control of the heart rate as a mechanism for the observed arrhythmias in pneumonia.

By unraveling the intricate connections between the lungs and the heart, the way for new insights, interventions, and ultimately, improved management of patients suffering from both pulmonary and cardiovascular conditions is paved.

Based on these data it is expected that administration of an anti-inflammatory, neuroprotective and/or cell death-preventing agent to the stellate ganglion will be clinically effective in preventing and/or treating cardiac dysfunction, particularly in a subject suffering from a respiratory disease.

In some embodiments, the cardiovascular dysfunction is selected from acute coronary syndrome, myocardial infarction, heart failure, endocarditis, myocarditis, pericarditis, venous thromboembolism, arrhythmia, blood pressure fluctuations such as hypotension and/or pulmonary hypertension. In particular embodiments, the cardiovascular dysfunction comprises arrhythmia.

In some embodiments, administration of the agent inhibits and/or prevents damage in the stellate ganglion of the subject, e.g., degeneration and/or death of cells in the stellate ganglion, particularly degeneration and/or death of neuronal cells.

For clinical use, particularly in preventive applications, the agent is administered in an effective dose to a subject suffering from a respiratory disorder associated with and/or accompanied by degeneration and/or cell death in the stellate ganglion. The subject can be any mammalian subject, typically a human subject.

The agent is administered to a subject suffering from a respiratory disorder. In some embodiments, the respiratory disorder is selected from pneumonia, ARDS, chronic bronchitis, or tuberculosis.

In some embodiments, the respiratory disorder is a pathogen-associated respiratory disorder including a virus- or bacteria-associated respiratory disorder e.g., pathogen-associated ARDS or pneumonia including virus- or bacteria-associated ARDS or pneumonia. In particular embodiments, the respiratory disorder is associated with a coronavirus, RSV, influenza, adenovirus, Haemophilus or Pneumococcus infection, e.g., with a SARS CoV-2 infection. For example, the respiratory disorder may be coronavirus-associated ARDS or pneumonia, e.g., a SARS CoV-2-associated ARDS or pneumonia.

In some embodiments, the respiratory disorder is a non-pathogen-associated respiratory disorder including a non-pathogen-associated pneumonia including COPD, asthma, and pulmonary fibrosis.

In some embodiments, the agent is administered to a subject who already suffers from a cardiovascular dysfunction as described herein. In some embodiments, the agent is administered to a subject who is at risk of acquiring a cardiovascular dysfunction as described herein. In some embodiments, the agent is administered to a subject who is characterized by having a damage in the stellate ganglion. In some embodiments, the agent is administered to a subject who has a substantially intact stellate ganglion.

In some embodiments, administration of the agent according to the present invention is particularly useful when the subject is suffering from an acute form of the respiratory disorder, e.g., when signs and/or symptoms of the disease are observable for a time period of up to 4 weeks, up to 1 week or up to 1 day.

The agent may be administered as a monotherapy or in combination with a further medicament, particularly with a medicament suitable for the prevention and/or treatment of the respiratory disorder and/or with medicament suitable for the prevention and/or treatment of a cardiac dysfunction. For example, the agent may be administered with any standard treatment, e.g., standard surgical procedure and/or standard medicament useful for the treatment of respective disorder. In certain embodiments, the agent is administered with any standard treatment for a cardiac dysfunction, e.g., a standard surgical procedure and/or standard medicament useful for the treatment of the respective cardiac dysfunction. In some embodiments, the agent is administered with any standard treatment for a respiratory disorder e.g., a standard surgical procedure and/or standard medicament useful for the treatment of the respective respiratory disorder.

In certain embodiments, the agent is administered as a preventive intervention. In these embodiments, the subject has a substantially intact stellate ganglion that, however, may be at risk of suffering damage.

In particular embodiments, the stellate ganglion may be characterized by substantially normal ganglion activity, e.g., absence of disrupted activity. Disrupted activity may be characterized by increased periodic repolarization dynamics, e.g., assessed by 3D high-resolution resting ECGs and/or by a decreased blood melatonin level. and/or inflammation at the start of treatment.

In further particular embodiments, the stellate ganglion may be characterized by absence of inflammation. Absence of inflammation may be characterized by increased inflammation parameters such as an increased number of pro-inflammatory immune cells, e.g., macrophages, as determined e.g., by molecular imaging modalities, including PET, MRI and optoacoustic methods (e.g., inflammation score).

The agent is typically administered to the subject as a pharmaceutical composition comprising the active agent and a pharmacologically acceptable carrier. The carrier may be any suitable pharmaceutical carrier. The agent is administered in a therapeutically effective dose which may be determined by the skilled practitioner. The pharmaceutical composition may be in the form of a solid dosage form, e.g., a tablet, a capsule etc. or a liquid dosage form, e.g., a solution, emulsion, suspension, or the like. In particular embodiments, the pharmaceutical composition is a liquid dosage form adapted for injection.

The agent or the pharmaceutical composition may be administered in any suitable way, e.g., locally, or systemically. The primary form of application of the agent hereby is the local injection. In particular embodiments, the agent is administered by ultrasound-guided injection into the stellate ganglia. Alternatively, systemic administration, including oral, parenteral, e.g., by subcutaneous injection, or intravenous infusion or injection, and transmucosal application, may be applied.

In some embodiments, the agent may be any pharmaceutically acceptable anti-inflammatory agent, particularly any pharmaceutically acceptable anti-inflammatory agent capable of counteracting inflammatory processes mediated by, and/or associated with pro-inflammatory cells, e.g., pro-inflammatory macrophages.

For example, the anti-inflammatory agent may be a steroidal drug, an immunoglobulin preparation, an inhibitor of the complement cascade, an immunomodulator, and/or an antagonist of a chemokine, cytokine, or colony-stimulating factor. In certain embodiments, the anti-inflammatory agent is a biological, e.g., a monoclonal antibody, a recombinant fusion protein or a nucleic acid therapeutic.

In certain embodiments, the anti-inflammatory agent may be selected from:
- a glucocorticoid, including but not limited to Dexamethasone;
- an immunoglobulin preparation, including but not limited to IVIg;
- a complement inhibitor, including but not limited to a C3 antagonist such as Compstatin or Pegcetacoplan, or a C5 antagonist such as Eculizumab, Zilucoplan, Tesidolumab, or SKY59;
- a nucleic acid therapeutic including but not limited to anti-miR21;
- an immunomodulator, including but not limited to Alemtuzumab, Mitoxantron, or Orcrelizumab; and
- an antagonist of a chemokine, cytokine or colony-stimulating factor including but not limited to Etanercept, Adalimumab or PLX5622;
- a macrophage inhibitor including but not limited to a bisphosphonate compound such as clodronate;
- a NLRP3 inflammasome inhibitor such as MCC950, Glyburide, Partehnolide, or β-OH-Butyrate; and
- a CSF1R inhibitor such as Pexidartinib.

In certain embodiments, a macrophage inhibitor such as clodronate is administered locally to a peripheral ganglion, e.g., the SCG, for example to a subject suffering from a pressure overload-induced cardiac dysfunction.

In some embodiments, the agent may be any pharmaceutically acceptable neuroprotective agent, particularly any pharmaceutically acceptable neuroprotective agent capable of counteracting neuronal damages mediated by, and/or associated with various factors such as axonal damage, oxidative stress, inflammation, excitotoxicity, or other pathological processes.

For example, the neuroprotective agent may be a free radical scavenger, nerve growth factor/neurotrophic factor, mitochondrial enhancer, or statin.

In certain embodiments, the neuroprotective agent may be selected from:
- a free radical scavenger including but not limited to NXY-059, PEG-SOD, Tempol, hydroxystilbene, and oxyresveratrol;
- a nerve growth fand/or neurotrophic factor including but not limited to an insulin-like growth factor (IGF), nerve growth factor (NGF) and a brain-derived neurotrophic factor (BDNF);
- a mitochondrial enhancer including but not limited to Carnitine, Resveratrol, PQQ and alpha-lipoic acid;
- a nucleic acid therapeutic; and
   - a statin including but not limited to Atorvastatin, Mevastatin, Rosuvastatin and Simvastatin.

In some embodiments, the agent may be any pharmaceutically acceptable cell death-preventing agent, particularly any pharmaceutically acceptable cell death-preventing agent capable of counteracting apoptotic processes mediated by, and/or associated with various factors such as axonal damage, oxidative stress, inflammation, excitotoxicity, or other pathological processes.

For example, the cell death-preventing agent may be a Bcl-2 family modulator or Caspase inhibitor.

In certain embodiments, the cell death-preventing agent is selected from:
- a Bcl-2 family modulator including but not limited to Venetoclax, Navitoclax, Obatoclax and ABT-199;
- a nucleic acid therapeutic; and
- a caspase inhibitor including but not limited to Z-VAD-FMK, Q-VD-OPh, Emricasan, VX-765 and Z-DEVD.

In certain embodiments, the active agent of the invention is a nucleic acid therapeutic, e.g., an antisense molecule, an RNAi agent such as an siRNA or a genome editing compound. The nucleic acid therapeutic may be adapted for targeting macrophages, e.g., by coupling to a ligand such a carbohydrate. In particular embodiments, the nucleic acid therapeutic administered locally to the stellate ganglion, e.g., by injection.

Still a further aspect of the present invention relates to a method for identifying a subject being at risk of a cardiovascular dysfunction, comprising determining at least one parameter associated with the function of the stellate ganglion. In particular embodiments, the subject suffers from a respiratory disease.

Particularly, this method is suitable for identifying a respiratory patient that is at risk for cardiac dysfunction. The identified subject may then be treated with an anti-inflammatory agent as described above. A subject "being at risk of damage" includes a subject that has not suffered any damage yet but is expected to suffer from damage including complete loss of ganglionic function if left untreated. It also includes a subject that has already suffered a certain degree of damage and is expected to suffer from increased damage including complete loss of ganglionic function if left untreated.

Identification of the subject may comprise determining at least one parameter associated with a risk for ganglion damage. In some embodiments, the parameter is selected from a physical parameter, and/or a physiological parameter and/or a biomarker, e.g., a biomarker generally associated with a cardiac disease, and/or a biomarker specifically associated with damage of the stellate ganglion.

In some embodiments, the parameter may be a physical parameter such as periodic repolarization dynamics, e.g., as assessed by a 3D high-resolution resting ECG, or ganglion size, e.g., as assessed by ultrasound. An aberrant periodic repolarization dynamics and/or ganglion hypertrophy may be associated with risk of damage.

Further, the parameter may be a physiological parameter such as ganglion function, inflammation and/or a biomarker associated with risk of ganglionic damage. For example, the biomarker may be a biomarker specifically associated with damage or risk of damage of the stellate ganglion. In some embodiments, the biomarker is a biomarker generally associated with a cardiac disease including but not limited to ANP, BNP, Troponin T and derivatives thereof.

In some embodiments, the biomarker is specifically associated with damage or risk of damage of the stellate ganglion. For example, the ganglion-specific biomarker is a gene, e.g., a human gene, which is characterized by an altered, e.g., increased, or decreased expression level associated with damage or risk of damage in the stellate ganglion.

In some embodiments, the biomarker is a long non-coding RNA (IncRNA) biomarker characterized by a cell-specific expression (macrophage specific) and disease-associated expression differences.

In some embodiments, the biomarker is a micro RNA biomarker, e.g., miR-21 which is the most abundant microRNA in cardiac and other macrophages and macrophage-specific genetic deletion of miR-21 prevents myocardial remodeling and dysfunction in a mouse model of heart failure (Ramanujam & Schön et al., Circ. 2021).

Given our findings that respiratory patients with associated cardiac dysfunction show a pronounced macrophage-driven inflammatory response in the stellate ganglion it is expected that deletion of macrophages protects against "ganglion disease" and cardiac dysfunction.

Still a further aspect of the present invention relates to a compound which is a modulator of a biomarker selected from a gene which is characterized by an altered, e.g., increased or decreased expression level associated with damage or risk of damage in the stellate ganglion for use in a method for preventing and/or treating cardiac dysfunction in a subject, particularly in a subject suffering from a respiratory disease.

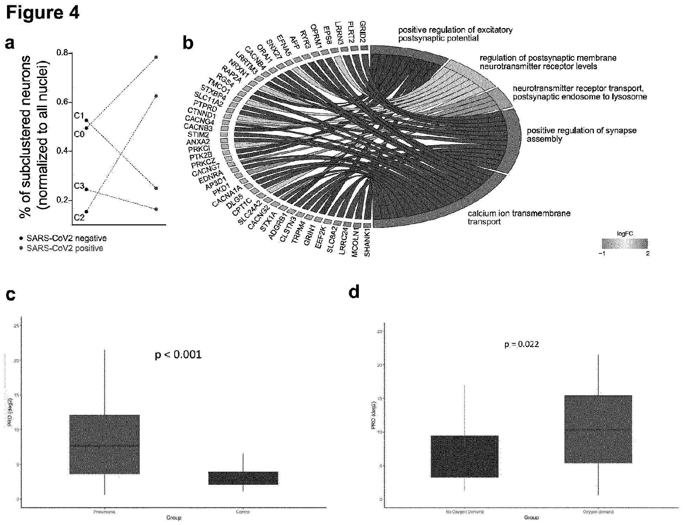

In particular embodiments, the gene is a human gene selected from GRID2, FLRT2, RRN3, EPS8, OPRM1, RYR3, APP, EFNA5, SNX27, ORAI1, CACNB4, LRRTM3, NRXN1, RAP2A, RGS4, TMCO1, STXBP4, SLC11A2, PTPRD, CTNND1, CACNG4, CACNB3, STIM2, ANXA2, PRKCI, and PTK2B, as described in Figure 4b or a mammalian ortholog thereof. These genes show an increased expression in subjects suffering from COVID pneumonia.

In particular embodiments, the gene is a human gene selected from PRKCZ, CACNG7, EDNRA, AP3D1, PKD1, CACNA1A, DLG5, CPT1C, SLC24A2, CACNG2, STX1A, ADGRB1, CLSTN3, TRPM4, GRIN1, EEF2K, SLC8A2, LRRC24, MCOLN1 and SHANK1 as described in Figure 4b or a mammalian ortholog thereof. These genes show a decreased expression in subjects suffering from COVID pneumonia.

Nucleotide sequences of the indicated human genes and transcripts thereof are disclosed in publicly available databases such as ENSEMBL. These nucleotide sequences in their versions from the filing date of the present application are herein incorporated by reference.

In some embodiments, the compound may be an agonist of the biomarker, particularly when a decreased expression level is associated with damage or risk of damage in a peripheral ganglion. In some embodiments, the compound may be an antagonist of the biomarker, particularly when an increased expression level is associated with damage or risk of damage in a peripheral ganglion. In some embodiments, an agonistic modulator may be the polypeptide including an active fragment or derivative thereof or an mRNA encoding such a polypeptide. In some embodiments, an antagonistic modulator may be an antibody or an antigen-binding fragment thereof or a nucleic acid molecule, e.g., an mRNA molecule, an antisense molecule, or a siRNA molecule capable of inhibiting gene expression. In some embodiments, the compound may be a genome editing compound, such as CRISP/Cas, a zinc finger nuclease (ZFN), or a transcription activator-like effector nuclease (TALEN), preferably selective gene silencing through application of the CRISPRi/dCas9 system. The CRISPR/Cas component may comprise a CRISPR enzyme, CRISPR RNA (crRNA) and transactivating crRNA (tracrRNA). Suitable CRISPR enzymes are selected from Cas enzymes, Cas nickases, catalytically inactive Cas variants, preferably dCas9 or alternatively dCas13.

The modulator may be administered as a pharmaceutical comprising the active agent and a pharmacologically acceptable carrier, e.g., as described above. In certain embodiments, the modulator is administered locally as described above. The modulator may be administered as a monotherapy or in combination with another medicament, particularly an anti-inflammatory agent as described above.

The present invention shall be explained in more detail by the following figures and examples.

### FIGURE LEGENDS

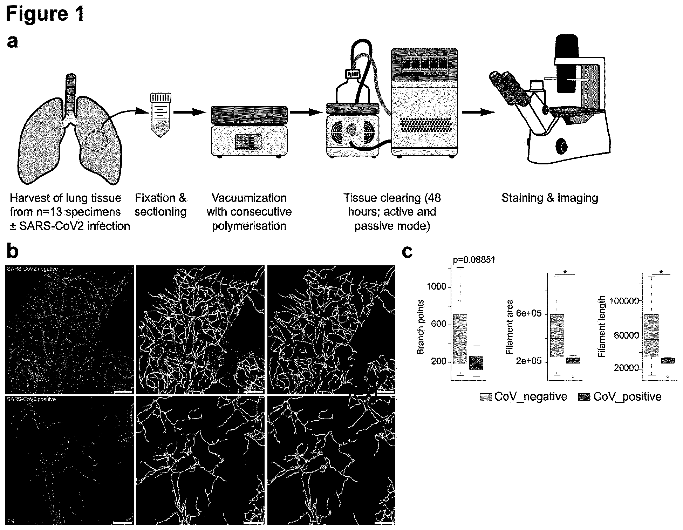

Figure 1. Infection with SARS-CoV2 causes impaired axonal innervation in the lung. (a) Schematic overview of experimental procedure. Lung tissue samples were first sliced in 1mm sections, followed by polymerization, tissue clearing, staining and imaging. (b) Representative overview images of cleared lung slices from specimens with and without SARS-CoV2 infection stained for tyrosine hydroxylase. Scale bar: 100 µm. (c) Quantification of filament area, length, and branching points. Data are mean with standard deviation from n=8 (CoV_negative) and n=5 (CoV_positive). Student's t-test was applied for statistical analysis. * P< 0.05.

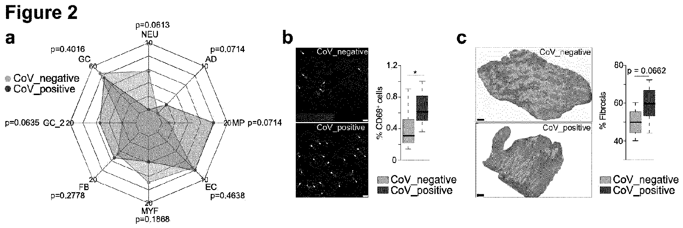

Figure 2. SARS-CoV2 infection changes the cellular composition in the stellate ganglion. (a) Quantitative assessment of cellular composition in stellate ganglia by genetic deconvolution of RNA sequencing libraries (% of relative fraction). (b) Left CD68+ cells (arrows) in the respective ganglia with right quantification. Scale bar: 50 µm. (c) Left Representative images of human stellate ganglia (SG) stained with Fast Green/Sirius Red. Scale bar: 200 µm. Right Quantification of SG fibrosis. Data are mean ± standard deviation from n=5 (CoV_negative) and n=4 (CoV_positive) for genetic deconvolution, n=9 (CoV_negative) and n=8 (CoV_positive) for macrophage quantification, n=8 (CoV_negative) and n=7 (CoV_positive) for quantification of fibrosis. Student's t-test or Mann Whitney test was applied for statistical analysis. * P< 0.05. Abbreviations: NEU - sympathetic neurons, AD - adiopocytes, MP - macrophages, EC - endothelial cells, MYF - myofibroblasts, FB - fibroblasts, GC - glial cells.

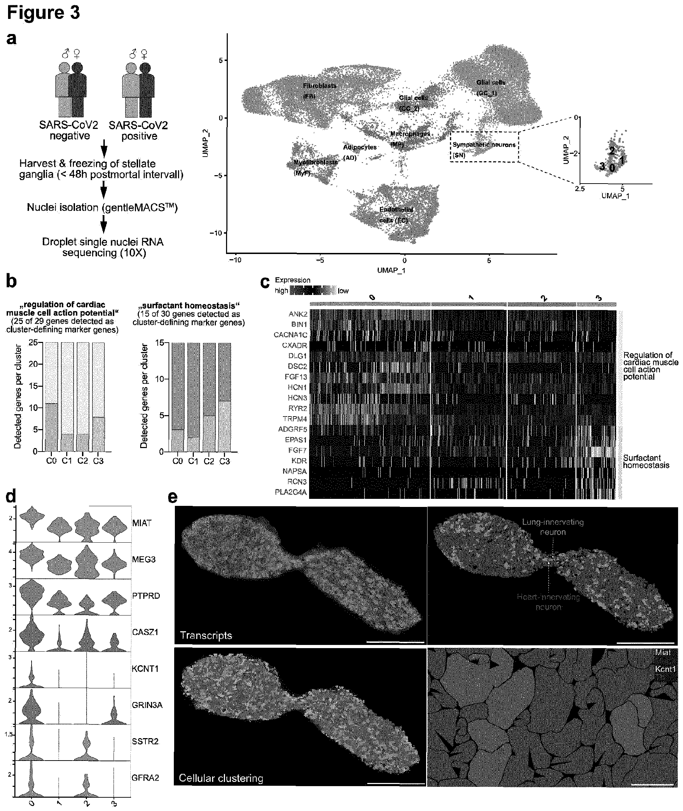

Figure 3. Identification and characterization of neuronal subsets in the stellate ganglion using single nuclei and spatial RNA sequencing. (a) Left Experimental workflow. Right UMAP projection of 35,297 cellular nuclei from SGs isolated from human specimens with and without infection with SARS-CoV2. (b) Detected marker genes from indicated gene ontology terms. (c) Heatmap of postulated genes involved in organ-specific neuronal signaling. (d) Violin plot of known marker genes of heart-innervating stellate neurons. (e) Left Representative overview of detected transcripts and cellular clusters in a murine stellate ganglion section. Right above Overview of location of heart- and lung-innervating neurons in the stellate ganglion. Right below Representative area in high magnification. Overlay shows the merging of cell segmentation and neuron subcluster-defining marker genes. Scale bar: 500 µm (close-up: 25 µm).
Figure 4. Heart-innervating stellate neurons are characterized by hyperactivity upon infection with SARS-CoV2. (a) Relative number of neuronal subcluster (in % to total nuclei). (b) GO term analysis of upregulated processes in the heart-innervating neuronal subcluster upon infection with SARS-CoV2 (c-d) ECG assessment of SARS-CoV2 positive and control patients. (c) Assessment of sympathetic activity, indicated by PRD (periodic repolarization dynamics). (d) Correlation of PRD to oxygen demand in patients.

### EXAMPLES

### 1. Results

### 1.1 Infection with SARS-CoV2 causes impaired axonal innervation in the lung.

SARS-CoV2 infection is associated with substantial pulmonary tissue damage that can ultimately lead to a highly lethal pneumonia (Melms et al., 2021). Despite its central role in controlling lung function, the pulmonary sympathetic innervation has - to the best of our knowledge - not been investigated with regard to pathological alterations due to COVID-19.

We therefore collected lung tissue from 13 specimens upon autopsy that were either tested positive or negative for SARS-CoV2. We then acquired 1 mm sections and performed optical clearing, followed by staining for the sympathetic marker tyrosine hydroxylase (TH; Fig. 1a). Comparative analysis suggested substantial loss of pulmonary sympathetic innervation, indicated by significantly reduced axonal area and length (Fig. 1b-c). This finding prompted us to next investigate the stellate ganglia (SG) from which the pulmonary sympathetic axons originate.

### 1.2 SARS-CoV2 infection changes the cellular composition in the stellate ganglion.

To this end, we prospectively collected SGs from a total of 17 specimens, 8 of which were tested positive for SARS-CoV2. In this postmortem study, male and female individuals were included and represented in an age-matched manner (study characteristics see supplementary figure Fig. S1). Using single nuclei RNA as well as deep RNA sequencing, we first created a signature matrix of marker genes specific for the major cell populations in the stellate ganglion. This matrix was then used as a basis for genetic deconvolution (Newman et al., 2015) of deep RNA sequencing data from human stellate ganglion libraries. Ganglia from humans that were tested positive for SARS-CoV2 displayed a marked increase of the macrophage, adipocyte and glia cell fraction, as well as a reduction of sympathetic neurons (Fig. 2a) while the (myo)fibroblast or endothelial cell fractions remained largely unchanged.

To independently validate the findings of changes in cellular composition, tissue cryosections of stellate ganglia were stained for the macrophage marker CD68 (antibody, Fig. 2b) and for Sirius Red/Fast Green (Fig. 2c). Quantitative analysis confirmed a prominent increase of CD68+ macrophages without a significant increase of interstitial fibrosis (Fig. 2b-c), suggesting a rather reversible ganglion damage.

### 1.3 Identification and characterization of neuronal subsets in the stellate ganglion using single nuclei and spatial RNA sequencing

To dissect and quantitatively assess the adaptive cellular changes upon SARS-CoV2 infection, we performed single nuclei RNA sequencing (snSeq) of human SGs (Fig. 3a). We sequenced in total four SGs, two of which were from human specimens tested positive for SARS-CoV2. Hereby, SGs with the shortest postmortem interval were chosen (< 24 hours). In total, 35,297 nuclei passed the quality control and were applied to the computational analysis. We were able to detect seven major cell types, namely sympathetic neurons, glial cells, (myo)fibroblasts, endothelial cells, adipocytes, and macrophages (Fig. 3b, Fig. S1a). Interestingly, cellular frequency drastically changed upon COVID-19, with the most prominent increase in glial cells and macrophages confirming our previous immunofluorescence-based quantification (Fig. S1b). Sub-setting and in-depth clustering of sympathetic neurons revealed four distinct subclusters of noradrenergic neurons (Fig. 3b *right*). As the stellate innervates a multiplicity of organs, amongst others the heart and the lung, we aimed to determine organ-specific subpopulations. Interestingly, comparison of cell cluster-defining marker genes with gene sets for organ-specific functions assessed by gene ontology (GO) showed a clear pattern with enrichment for heart-related processes in cluster 0, while lung-related processes (surfactant homeostasis) was enriched in cluster 3 (Fig. 3b-c).Additionally, we used marker genes from state-of-the-art retrograde tracing studies and were able to confirm the identity of cluster 0 neurons as a heart-innervating subset of sympathetic neurons (Fig. 3d). Next, we performed spatial RNA sequencing in order to validate the location of heart- and lung-innervating neurons in the stellate ganglion. Surprisingly, both neuronal subpopulations seem to be located in close proximity to each other (Fig. 3e).

### 1.4 Heart-innervating stellate neurons are characterized by hyperactivity upon infection with SARS-CoV2.

Quantitative analysis of neuronal subclusters indicated a relative shift upon SARS-CoV2 infection, with a relative increase of the heart-innervating cluster (C0, Fig. 4a). Differential expression analysis in this cluster between SARS-CoV negative and positive conditions revealed significant functional alterations, mainly regarding synaptic transmission. In particular, GO term analysis suggested increased postsynaptic potential, neurotransmitter receptor levels, synapse assembly, and calcium ion transport (Fig. 4b). To validate the clinical relevance of this finding, we then performed a prospective clinical study and assessed sympathetic activity from ECG recordings. The results indeed show an increased sympathetic activity in SARS-CoV2-infected patients compared to control patients (Fig. 4c). Increased PRD but lower than official cut-off for CVD. This effect was also high dependent on oxygen demand (Fig. 4d), indicating a higher sympathetic tone in these patients.

### 2. Discussion

The present inventors have identified coincidence of a marked injury of lung-innervating axons with macrophage accumulation and increased neuronal activity in heart-innervating sympathetic neurons.

These findings highlight the importance of specialized, organ-innervating subsets of sympathetic neurons and their spatial integration within the stellate ganglion, which provides innervation to the heart and lung. Intriguingly, maladaptive ganglionic remodeling was observed in pneumonia, independent of the causal origin. Assuming comparable degrees of ganglionic activation and inflammation, we assume that the discriminating factor is the extent of neuronal injury in disease. Thus, the further systematic investigation of the reversibility of ganglionic disease appears warranted.

In the light of potential treatment strategies, preventive anti-inflammatory interventions seem to be favorable and might attenuate neuronal dysfunction. While not directly tested in the present study, this would be in line with previous studies in CNS disease, suggesting that therapeutic interference with microglia (central nervous system analogs to macrophages) activation may promote neuronal survival and rescue neuronal function (Qin et al., 2021; Dreismann et al., 2021; Absinta et al. 2021; Gavriilaki et at., 2020; Matheis et al.,2020).

Furthermore, understanding the time course of lung disease and the optimal treatment timepoint, particularly for chronic diseases such as COPD, remains to be determined. The investigation of lung-innervating axon injury, macrophage accumulation, and heightened neuronal activity in heart-innervating sympathetic neurons presents a promising avenue for further research. Understanding the sequence of events leading to the onset of cardiac arrhythmias during lung disease could provide valuable insights into therapeutic interventions and preventative strategies.

Ultimately, this study is in line with previous application PCT/EP2023/065279 showing a disease-spreading through the integration of organ-specific neuronal subpopulations in peripheral sympathetic ganglia. By uncovering the intricate connections between the lungs and the heart at the neuronal level, we move closer to understanding the pathogenesis of respiratory-related cardiac complications. Further research in this field holds promise for the development of targeted interventions and improved management strategies, ultimately benefiting patients and providing valuable treatment modalities.

### REFERENCES

Absinta, Martina et al. 2021. "A Lymphocyte-Microglia-Astrocyte Axis in Chronic Active Multiple Sclerosis." Nature 597(September).
Azevedo, Rafael Bellotti et al. 2021. "Covid-19 and the Cardiovascular System: A Comprehensive Review." Journal of Human Hypertension 35(1): 4-11.
Bartlett, Benjamin et al. 2022. "Cardiovascular Changes after Pneumonia in a Dual Disease Mouse Model." Scientific Reports 12(1): 1-10.
Bartlett, Benjamin, Herbert P. Ludewick, Silvia Lee, and Girish Dwivedi. 2019. "Cardiovascular Complications Following Pneumonia: Focus on Pneumococcus and Heart Failure." Current Opinion in Cardiology 34(2): 233-39.
Corrales-Medina, Vicente F. et al. 2011. "Cardiac Complications in Patients with Community-Acquired Pneumonia: A Systematic Review and Meta-Analysis of Observational Studies" ed. Keith P. Klugman. PLoS Medicine 8(6): e1001048.
Corrales-Medina, Vicente F. et al. 2012. "Cardiac Complications in Patients with Community-Acquired Pneumonia Incidence, Timing, Risk Factors, and Association with Short-Term Mortality." Circulation 125(6): 773-81.
Corrales-Medina, Vicente F., Daniel M. Musher, Svetlana Shachkina, and Julio A. Chirinos. 2013. "Acute Pneumonia and the Cardiovascular System." The Lancet 381(9865): 496-505.
Dreismann, Anna K. et al. 2021. "Functional Expression of Complement Factor I Following AAV-Mediated Gene Delivery in the Retina of Mice and Human Cells." Gene Therapy 28(5): 265-76.
Gavriilaki, Maria, Vasilios K. Kimiskidis, and Eleni Gavriilaki. 2020. "Precision Medicine in Neurology: The Inspirational Paradigm of Complement Therapeutics." Pharmaceuticals 13(11): 1-25.
Herring, Neil, Manish Kalla, and David J. Paterson. 2019. "The Autonomic Nervous System and Cardiac Arrhythmias: Current Concepts and Emerging Therapies." Nature Reviews Cardiology (Mi).
Lee, Kun-Yu et al. 2022. "Risk of Atrial Fibrillation in Patients with Pneumonia." Heart & Lung 52: 110-16.
Matheis, Fanny et al. 2020. "Adrenergic Signaling in Muscularis Macrophages Limits Infection-Induced Neuronal Loss." Cell 180(1): 64-78.e16.
Melms, Johannes C. et al. 2021. "A Molecular Single-Cell Lung Atlas of Lethal COVID-19." Nature 595(7865): 114-19.
Newman, Aaron M. et al. 2015. "Robust Enumeration of Cell Subsets from Tissue Expression Profiles." Nature Methods 12(5): 453-57.
Pletz, Mathias W. et al. 2022. "Unmet Needs in Pneumonia Research: A Comprehensive Approach by the CAPNETZ Study Group." Respiratory Research 23(1).
Qin, Delong et al. 2021. "Traumatic Brain Injury: Ultrastructural Features in Neuronal Ferroptosis, Glial Cell Activation and Polarization, and Blood-Brain Barrier Breakdown." Cells 10(5).
Ramanujam, Deepak et al. 2021. "MicroRNA-21-Dependent Macrophage-to-Fibroblast Signaling Determines the Cardiac Response to Pressure Overload." Circulation 143(15): 1513-25.
Stotts, Cameron, Vicente F. Corrales-Medina, and Katey J Rayner. 2023. "Pneumonia-Induced Inflammation, Resolution and Cardiovascular Disease: Causes, Consequences and Clinical Opportunities." Circulation Research 132(6): 751-74.
Thomson, K.Jefferson, David D. Rutstein, Daniel M. Tolmach, and William H. Walker. 1946. "Electrocardiographic Studies during and after Pneumococcus Pneumonia." American Heart Journal 31(5): 565-79.
Végh, Anna et al. 2016. "Part and Parcel of the Cardiac Autonomic Nerve System: Unravelling Its Cellular Building Blocks during Development." Journal of Cardiovascular Development and Disease 3(3): 28.

## Claims

1. An agent selected from the group consisting of an anti-inflammatory agent, a neuroprotective agent, and a cell death-preventing agent for use in a method for preventing and/or treating cardiovascular dysfunction in a subject suffering from a respiratory disease.

2. The agent of claim 1 for the use of claim 1, wherein the cardiovascular dysfunction is selected from acute coronary syndrome, myocardial infarction, heart failure, endocarditis, myocarditis, pericarditis, venous thromboembolism, arrhythmia, and blood pressure fluctuations such as hypotension and/or pulmonary hypertension.

3. The agent of claim 1 for the use of claim 1 or 2, wherein the cardiovascular dysfunction comprises arrhythmia.

4. The agent of claim 1 for the use of any one of claims 1-3, wherein the inhibits and/or prevents damage in the stellate ganglion of the subject.

5. The agent of claim 1 for the use of any one of claims 1-4, wherein the agent inhibits and/or prevents degeneration and/or death of cells in the stellate ganglion, particularly degeneration and/or death of neuronal cells.

6. The agent of claim 1 for the use of any one of claims 1-5, wherein the subject is suffering from a respiratory disorder selected from acute respiratory distress syndrome (ARDS), pneumonia, chronic bronchitis, or tuberculosis.

7. The agent of claim 1 for the use of any one of claims 1-6, wherein the subject is suffering from pathogen-associated a pathogen-associated respiratory disorder including virus- or bacteria-associated ARDS or pneumonia, particularly from coronavirus-associated ARDS or pneumonia.

8. The agent of claim 1 for the use of any one of claims 1-7, wherein the subject is suffering from an acute form of the respiratory disorder.

9. The agent of claim 1 for the use of any one of claims 1-8, wherein the agent is administered as a preventive intervention.

10. The agent of claim 1 for the use of any one of claims 1-8, wherein the agent is administered in response to an observed damage and/or dysfunction of the stellate ganglion.

11. The agent of claim 1 for the use of any one of claims 1-10, wherein the agent is locally administered to the stellate ganglion, e.g., by injection.

12. The agent of claim 1 for the use of any one of claims 1-11, wherein the agent is
(i) an anti-inflammatory agent particularly selected from:
- a glucocorticoid;
- an immunoglobulin preparation;
- a complement inhibitor;
- a nucleic acid therapeutic;
- an immunomodulator;
- an antagonist of a chemokine, cytokine or colony-stimulating factor;
- a macrophage inhibitor;
- a NLRP3 inflammasome inhibitor; and
- a CSF1R inhibitor;
(ii) a neuroprotective agent particularly selected from:
- a free radical scavenger;
- a nerve growth factor/neurotrophic factor;
- a mitochondrial enhancer;
- a nucleic acid therapeutic; and
- a statin;
(iii) a cell death-preventing agent particularly selected from:
- a Bcl2 family modulator;
- a nucleic acid therapeutic; and
- a caspase inhibitor.

13. A method for identifying a subject being at risk of a cardiovascular dysfunction, comprising determining at least one parameter associated with the function of the stellate ganglion.

14. A compound which is a modulator of a biomarker selected from a gene which is **characterized by** an altered, e.g., increased, or decreased expression level associated with damage or risk of damage in the stellate ganglion, for use in a method for preventing and/or treating cardiac dysfunction in a subject.

15. The compound of claim 14 for the use of claim 14,
(i) which is an agonist of the biomarker or an antagonist of the biomarker, and/or
(ii) which is selected from an antibody or an antigen-binding fragment thereof and a nucleic acid molecule, e.g., an mRNA molecule, an antisense molecule, or a siRNA molecule, and/or
(iii) which is a genome editing compound, such as CRISP/Cas, a zinc finger nuclease (ZFN), or a transcription activator-like effector nuclease (TALEN), preferably a CRISPRi/dCas9 system for selective gene silencing.
